# EUROPEAN PATENT APPLICATION

(11) **EP 2 848 231 A1**
(43) Date of publication of application: **18.03.2015**
(21) Application number: 13788363.3
(22) Date of filing: 10.05.2013
(51) Int. Cl.: A61F 2/02, A61L 27/14

(54) **FILLER FOR REMOVING WRINKLES**

(30) Priority: 10.05.2012 KR 20120049953
(71) Applicant: Pacificpharma Corporation, Gangnam-gu, Seoul 135-733 (KR); HB Medicals, Co., Ltd., Yeonsu-gu Incheon 406-840 (KR); Lee, Hoon Bum, Gangnam-gu, Seoul 135-776 (KR)
(72) Inventor: LEE, Hoon Bum, Seoul 135-776 (KR); PARK, Won Seok, Yongin-si Gyeonggi-do 446-729 (KR); KIM, Hyuk, Yongin-si Gyeonggi-do 446-729 (KR); YOON, Ho Sang, Seoul 135-733 (KR); LEE, Jang Yeon, Seoul 135-733 (KR)
(74) Representative: Bugnion Genève
(86) International application number: PCT/KR2013/004168
(87) International publication number: WO 2013/169075

(57) **Abstract**

The present invention relates to a filler for removing wrinkles, comprising: a slim and long tubular main body penetrating through the subcutaneous tissue; and a through-hole provided so as to enable the cells of the peripheral tissue of the main body to move to the inside of the main body so as to form a fibrous tissue, the through-hole being formed so as to communicate with a hollow portion formed inside the main body in the longitudinal direction from the outer peripheral surface of the main body. The filler for removing wrinkles according to the present invention is not deformed or moved by the pressure of a skin or external forces after being inserted into the subcutaneous tissue, and can be applied to various body parts having wrinkles, including body parts having deep wrinkles so as to achieve (semi)permanent effects.

## Description

### [Technical Field]

This disclosure relates to a filler for removing wrinkles, and more particularly, to a filler for removing wrinkles, which is inserted into the hypoderm at wrinkle portions by means of plastic surgery to guide formation of new fibrous tissues, in which a long gap or through hole in a longitudinal direction is formed at one side of a hollow long tube with good elasticity so that a passage of fibroblast, blood platelet, macrophage, white blood cells, red blood cells or the like for forming fibrous tissues is formed in the entire filler.

### [Background Art]

With advancing years, wrinkles are formed at the skin surface of the face or body of a human. Wrinkles are mainly caused by shrinkage of muscles and drooping of the skin and fat layer due to gravity. Wrinkles are formed perpendicular to a shrinking direction of the muscle and become deeper with age.

In order to remove wrinkles, there is frequently used a method for paralyzing the muscle with Botox to eliminate the cause of wrinkles. However, Botox results in unnatural expression and is not be easily used to deep wrinkles as well as a portion under the eye, the lower lip, nasolabial folds or the like. In addition, the effect of Botox lasts just three to six months.

As another solution, a filler for inflating a depressed portion may be used. However, even though a liquid filler is conveniently injected, after being injected into the skin, the liquid filler moves in a shrinking direction of the muscle whenever the muscle moves, which makes the depressed portion to be looked relatively deeper, which is against its original purpose. In addition, the liquid filler is decomposed and absorbed after a certain period (1 to 2 years at the maximum).

As another solution, a very thin gold thread may be inserted into the hypoderm. The gold thread causes a foreign body reaction to make new tissues around the gold thread. However, the gold thread is not useful for deep wrinkles, and the inserted gold thread may be bent or protrude out of the skin due to an external force. In addition, the gold thread is left permanently and may give interference at X-ray, CT or MRI scanning, which may cause a problem in diagnosis.

### [Disclosure]

### [Technical Problem]

This disclosure is directed to providing a filler for removing wrinkles, which does not use skin incision and also does not move regardless of shrinkage of the muscle after being inserted into the hypoderm.

In addition, the present disclosure is directed to providing a filler for removing wrinkles, which guides formation of fibrous tissues including new collagen fibril in an entire region of a long tube serving as a surgical spot through a longitudinal cleft or through hole serving as a long gap formed along the entire length of the wrinkle-removing filler having a long tube shape, after being inserted into the hypoderm, so that dense fibrous tissues are formed rapidly and the surgery effect lasts (semi)permanently by maintaining the newly formed fibrous tissues even though the filler is decomposed and absorbed as time goes.

Moreover, the present disclosure is directed to providing a filler for removing wrinkles, which may be used for various wrinkle portions such as deep wrinkles and minimize a deformation caused by a pressure or external force applied to the skin after being inserted into the hypoderm by further including an elastic support with a recoiling force against an external force.

### [Technical Solution]

In one general aspect of the preset disclosure, there is provided a filler for removing wrinkles, which includes: an elongated integrated tubular main body having a hollow formed therein and allowing surgery through the hypoderm; and a longitudinal cleft formed through the main body in the length direction to form a moving passage of cells placed at neighboring tissues of the main body so that the cells move into the main body and form fibrous tissues.

In another aspect of the preset disclosure, there is provided a filler for removing wrinkles, which includes: an elongated integrated tubular main body having a hollow formed therein and allowing surgery through the hypoderm; and separated longitudinal clefts formed through the main body in the length direction and spaced apart from each other with at least two gaps to form a moving passage of cells placed at neighboring tissues of the main body so that the cells move into the main body and form fibrous tissues.

In another aspect of the preset disclosure, there is provided a filler for removing wrinkles, which includes: an elongated tubular main body having at least two rows of band-type partial bodies spirally rolled alternately; and a longitudinal cleft formed through the main body in the length direction.

In another aspect of the preset disclosure, there is provided a filler for removing wrinkles, which includes: a short and thin tubular main body having a hollow formed therein; a longitudinal cleft formed through the main body in the length direction from an outer circumference of the main body to an inner circumference thereof; and a connector installed in a plurality of short and thin tubular main bodies arranged to have hollows adjacent to each other so that the hollows are connected through the main body in the length direction.

In another aspect of the preset disclosure, there is provided a filler for removing wrinkles, which includes: an elongated integrated tubular main body having a hollow formed therein; a longitudinal cleft formed through the main body in the length direction; and an elastic cog formed by partially cutting a surface of the main body at an outer circumference thereof.

In another aspect of the preset disclosure, there is provided a filler for removing wrinkles, which includes: an elongated integrated tubular main body; a longitudinal cleft formed through the main body in the length direction; and an elastic cog formed by partially cutting a surface of the main body at an outer circumference thereof to have an acute angle with a decalcomanias shape symmetric based on a center portion of the main body.

In another aspect of the preset disclosure, there is provided a filler for removing wrinkles, which includes: an elongated integrated tubular main body having a hollow formed therein; separated longitudinal clefts formed through the main body in the length direction and spaced apart from each other with at least two gaps to form a moving passage of cells placed at neighboring tissues of the main body so that the cells move into the main body and form fibrous tissues; and an elastic cog formed by partially cutting a surface of the main body at an outer circumference thereof.

In another aspect of the preset disclosure, there is provided a filler for removing wrinkles, which includes: an elongated tubular main body having at least two rows of band-type partial bodies spirally rolled alternately; and a plurality of through holes formed through outer and inner surfaces of the main body, wherein the through holes are formed between the partial bodies.

In addition, in an exemplary embodiment of the present disclosure, the filler for removing wrinkles may further include a plurality of barriers formed in the hollow of the main body.

In addition, in an exemplary embodiment of the present disclosure, the filler for removing wrinkles may further include a spiral elastic support formed in the hollow of the main body.

In addition, in an exemplary embodiment of the present disclosure, the concave portions may be formed by shrinking the main body.

In an exemplary embodiment of the present disclosure, the main body may have a circular cross section with a diameter of 0.6 to 3.0 mm, and the hollow may have a diameter of 0.5 to 2.8 mm.

In addition, in an exemplary embodiment of the present disclosure, the longitudinal cleft may have a gap distance of 0.1 to 2.0 mm or a gap distance which is 1/8 to 1/4 of a circularly-converted circumferential length of a section of the main body.

In addition, in an exemplary embodiment of the present disclosure, the main body may have at least one through hole formed through the entire surface thereof.

In addition, in an exemplary embodiment of the present disclosure, the through hole may have a diameter of 40 to 500 µm in case of having a circular shape or a circularly-converted diameter of 40 to 500 µm with the same sectional area.

In addition, in an exemplary embodiment of the present disclosure, a contrast medium may be partially or entirely coated to an outside of the main body or included therein.

In addition, in an exemplary embodiment of the present disclosure, a growth factor for promoting formation of fibrous tissues may be coated to an outer circumference or inner circumference of the main body or included therein.

### [Advantageous Effects]

The filler for removing wrinkles according to the present disclosure may prevent the filler from being deformed or moving due to a pressure or external force applied to the skin after being inserted into the hypoderm since it has a recoiling force. In addition, since fibrous tissues including a large amount of new collagen fibril may be formed in a hollow of the main body through a longitudinal cleft or through hole provided in the outer circumference of the main body, the filler may be applied to various wrinkle portions such as deep wrinkles by adjusting a diameter of the main body, the hollow, the through hole or the like of the filler depending on the size and kind of the wrinkles, and also the newly formed fibrous tissues are maintained to last the wrinkle-removing effect (semi)permanently.

### [Description of Drawings]

Fig. 1 is a perspective view showing a filler for removing wrinkles having a longitudinal cleft according to a first embodiment of the present disclosure.
Fig. 2 is a perspective view showing a filler for removing wrinkles having a plurality of separated longitudinal clefts according to a second embodiment of the present disclosure.
Fig. 3 is a cross-sectional view showing a filler for removing wrinkles having a barrier in a hollow according to a third embodiment of the present disclosure.
Fig. 4 is a cross-sectional view showing a filler for removing wrinkles having a spiral elastic support in a hollow according to a fourth embodiment of the present disclosure.
Fig. 5 is a perspective view showing a filler for removing wrinkles, in which at least two rows of band-type partial bodies are alternately entangled according to a fifth embodiment of the present disclosure.
Fig. 6 is a cross-sectional view showing that fibrous tissues T are formed in a hollow of the filler for removing wrinkles according to the present disclosure, which is inserted into the hypoderm.
Fig. 7 is a perspective view showing a filler for removing wrinkles, in which a plurality of short main bodies respectively having a longitudinal cleft are interconnected by a connector according to a sixth embodiment of the present disclosure.
Fig. 8 is a cross-sectional view showing that the filler for removing wrinkles according to the sixth embodiment of the present disclosure is inserted into the hypoderm for plastic surgery.
Fig. 9 is a perspective view showing a filler for removing wrinkles, in which an elastic cog inclined to a side is formed at a surface of the main body according to a seventh embodiment of the present disclosure.
Fig. 10 is a cross-sectional view showing a plastic surgery state while the filler for removing wrinkles according to the seventh embodiment of the present disclosure is being inserted into the hypoderm.
Fig. 11 is a cross-sectional view showing that an elastic cog is hooked to the fibrous tissues T after the filler for removing wrinkles according to the seventh embodiment of the present disclosure is completely inserted into the hypoderm.
Fig. 12 is a perspective view showing a filler for removing wrinkles, in which an elastic cog having a decalcomanias shape symmetric based on a center portion of the main body is formed at a surface of the main body according to an eighth embodiment of the present disclosure.
Fig. 13 is a perspective view showing a filler for removing wrinkles, in which separated longitudinal clefts and an elastic cog are formed together according to a ninth embodiment of the present disclosure.
Fig. 14 is a cross-sectional view showing a plastic surgery state while the filler for removing wrinkles according to the ninth embodiment of the present disclosure is being inserted into the hypoderm.
Fig. 15 is a cross-sectional view showing that an elastic cog is hooked to the fibrous tissues T after the filler for removing wrinkles according to the ninth embodiment of the present disclosure is completely inserted into the hypoderm.
Fig. 16 is a perspective view showing a filler for removing wrinkles, in which a longitudinal cleft and a through hole are formed together according to a tenth embodiment of the present disclosure.
Fig. 17 is a perspective view showing a filler for removing wrinkles, in which separated longitudinal clefts and a through hole are formed together according to an eleventh embodiment of the present disclosure.
Fig. 18 is a perspective view showing a filler for removing wrinkles, in which a longitudinal cleft, a barrier and a through hole are formed together according to a twelfth embodiment of the present disclosure.
Fig. 19 is a perspective view showing a filler for removing wrinkles, in which a longitudinal cleft, a spiral elastic support and a through hole are formed together according to a thirteenth embodiment of the present disclosure.
Fig. 20 is a perspective view showing a filler for removing wrinkles, in which a plurality of short main bodies respectively having a longitudinal cleft and a through hole together are interconnected by a connector according to a fourteenth embodiment of the present disclosure.
Fig. 21 is a perspective view showing a filler for removing wrinkles, in which a longitudinal cleft, an elastic cog and a through hole are formed together according to a fifteenth embodiment of the present disclosure.
Fig. 22 is a perspective view showing a filler for removing wrinkles, in which separated longitudinal clefts, an elastic cog and a through hole are formed together according to a sixteenth embodiment of the present disclosure.
Fig. 23 is a perspective view and a partially enlarged view showing a filler for removing wrinkles, in which at least two rows of band-type partial bodies are alternately entangled according to a seventeenth embodiment of the present disclosure.

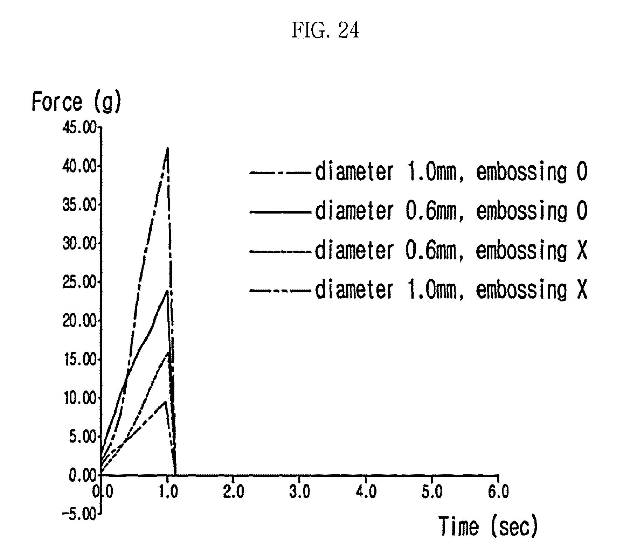

Fig. 24 is a graph showing a strength comparison experiment result for the filler for removing wrinkles according to the seventeenth embodiment of the present disclosure.
Fig. 25 is a diagram showing that the filler for removing wrinkles according the present disclosure is coupled to a needle.

### [Best Mode]

A filler for removing wrinkles according to the present disclosure includes: an elongated integrated tubular main body having a hollow formed therein and allowing surgery through the hypoderm; and a longitudinal cleft formed through the main body in the length direction to form a moving passage of cells placed at neighboring tissues of the main body so that the cells move into the main body and form fibrous tissues.

### [Mode for Invention]

Hereinafter, embodiments of the present disclosure will be described in detail with reference to the accompanying drawings.

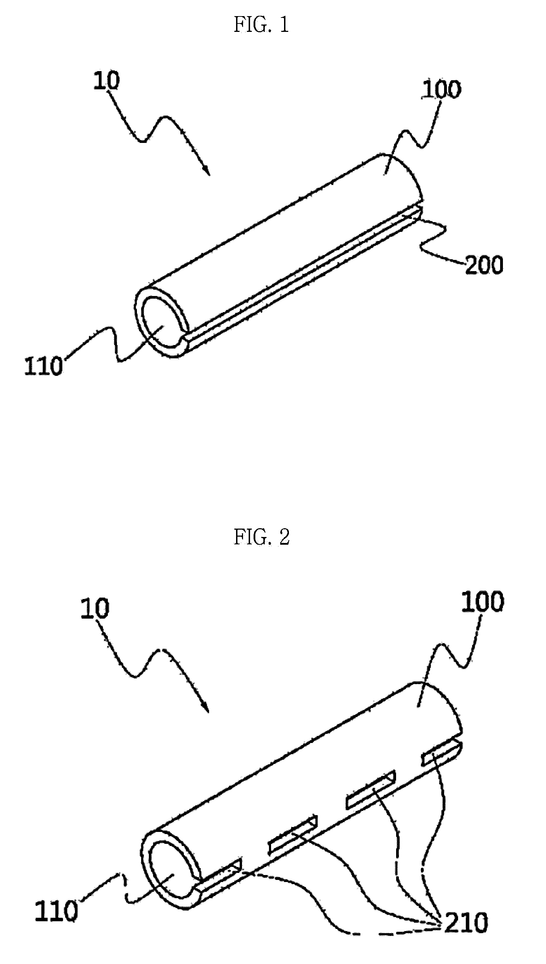

Fig. 1 is a perspective view showing a filler for removing wrinkles (or, also referred to as a wrinkle removing filler) having a longitudinal cleft according to a first embodiment of the present disclosure.

As shown in Fig. 1, the wrinkle removing filler 10 according to the first embodiment of the present disclosure includes an elongated integrated tubular main body 100 having a hollow formed therein and allowing surgery through the hypoderm, and a longitudinal cleft 200 formed through the main body 100 in the length direction to form a moving passage of cells placed at neighboring tissues of the main body so that the cells move into the main body 100 and form fibrous tissues.

At this time, the hollow 110 serving as an inner space of the main body gives a space in which cells come and form fibrous tissues, and is formed to communicate with external tissues of the main body through the longitudinal cleft 200. In addition, the longitudinal cleft 200 represents a long gap formed at the main body in the length direction. As the longitudinal cleft 200 is formed in the length direction of the main body as described above, fibrous tissues may be regenerated at a regular rate on the entire main body.

Fig. 2 is a perspective view showing a filler for removing wrinkles having a plurality of separated longitudinal clefts according to a second embodiment of the present disclosure.

As shown in Fig. 2, the wrinkle removing filler 10 according to the second embodiment of the present disclosure includes an elongated integrated tubular main body 100 having a hollow formed therein and allowing surgery through the hypoderm, and separated longitudinal clefts 210 formed through the main body in the length direction and spaced apart from each other with at least two gaps to form a moving passage of cells placed at neighboring tissues of the main body so that the cells move into the main body 100 and form fibrous tissues. At this time, the separated longitudinal clefts 210 are substantially identical to the longitudinal cleft 200 except that short gaps for allowing cells to move into the main body are formed at regular intervals.

In the filler for removing wrinkles according to the first and second embodiments of the present disclosure, the main body 100 may have a circular or polygonal (e.g., rectangular, hexagonal, octagonal or the like) cross section. More particularly, the main body 100 has a circular shape so as to be easily inserted into the hypoderm by threading at a needle (for example, a needle for plastic surgery) or moving along an intravenous injection needle inserted into a tube and moving through the hypoderm.

In addition, the main body 100 may have a diameter of 0.6 to 3.0 mm so as to be easily inserted into the hypoderm for surgery and located between the subcutaneous layer and the dermis layer or at the fat layer during the insertion surgery. Meanwhile, the main body 100 may have a suitable length depending on a surgery range.

Moreover, the hollow 110 formed in the main body 100 in the length direction gives a space in which elastic fibrous tissues and fibrous tissues such as collagen tissues may be newly formed by introducing fibroblast or the like of neighboring tissues through the longitudinal cleft 200. Therefore, if the diameter of the hollow 110 increases, an amount of newly formed fibrous tissues increases, and if the diameter of the hollow 110 decreases, the amount of newly formed fibrous tissues decreases. Therefore, the diameter of the hollow 110 may be suitably adjusted according to depth, location or the like of wrinkles, and is desirably 0.5 to 2.8 mm.

In addition, the longitudinal cleft 200 gives a passage through which fibroblast or the like may enter the hollow 110, and may have any shape such as circular, triangular, rectangular, octagonal, trapezoidal, or diamond shape. At this time, the longitudinal cleft 200 has a gap distance of 0.1 to 2.0 mm or 1/8 to 1/4 of a circularly-converted circumferential length of a section of the main body. If the diameter is too great, a supporting force against neighboring tissues is lost disadvantageously.

The main body 100 may be made of elastic material, or may be made of a biodegradable polymer such as hyaluronic acid (HA), polylactic acid (PLA), polyglyco-lactic acid (PGLA) and polydioxanone (PDS) or a non-biodegradable polymer such as nylon, silicon, polypropylene, polypropylethylene and Teflon. If a biodegradable polymer is used, the filler for removing wrinkles is slowly decomposed and absorbed after fibrous tissues are formed. If a non-biodegradable polymer is used, the filler for removing wrinkles permanently remains in the hypoderm.

In addition, the main body 100 may have a double-layer structure in which inner and outer layers of the main body are made of different materials. For example, the inner layer may be made of hard material, and the outer layer may be made of soft material. As an alternatively, the inner layer may be made of absorptive material, and the outer layer may be made of non-absorptive material. In addition, the inner and outer layers may be made of absorptive materials with different absorption rates.

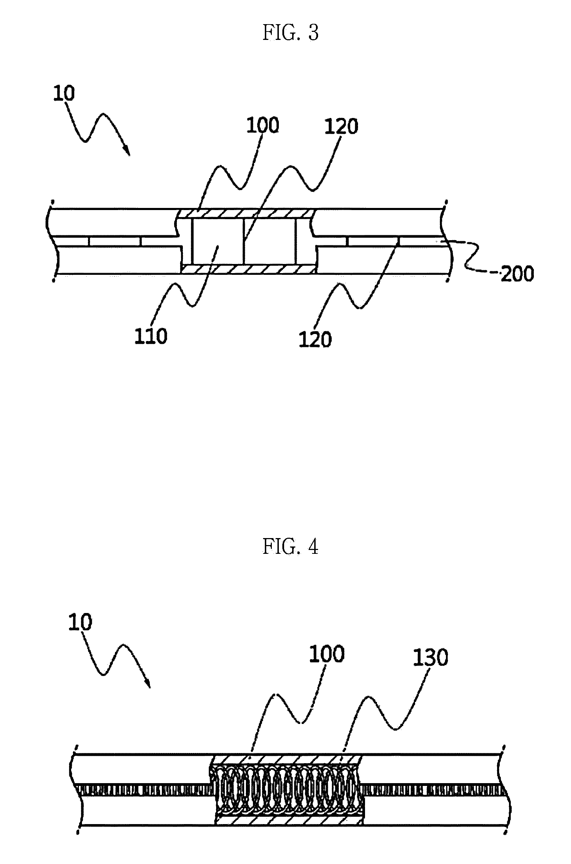

Fig. 3 is a cross-sectional view showing a filler for removing wrinkles having a barrier in a hollow according to a third embodiment of the present disclosure.

As shown in Fig. 3, the wrinkle removing filler 10 according to third embodiment of the present disclosure includes an elongated integrated tubular main body 100, a longitudinal cleft 200 formed through the main body 100 in the length direction, and a plurality of barriers 120 formed in the hollow 110 of the main body.

At this time, the barrier 120 gives a recoiling force so that the filler may maintain its shape without collapsing due to a pressure or external force applied to the skin after being inserted into the hypoderm, thereby reinforcing a supporting force of the main body.

The barrier 120 may be formed to entirely divide the hollow 110 of the main body or fill just a partial section of the hollow 110. In addition, the barrier 120 may be made of the same material as the main body 100.

Fig. 4 is a cross-sectional view showing a filler for removing wrinkles having a spiral elastic support in a hollow according to a fourth embodiment of the present disclosure.

As shown in Fig. 4, the wrinkle removing filler 10 according to the fourth embodiment of the present disclosure includes an elongated integrated tubular main body 100, a longitudinal cleft 200 formed through the main body 100 in the length direction, and a spiral elastic support 130 formed in the hollow 110 of the main body.

At this time, the spiral elastic support 130 may restore its original shape by its restoring force in the hollow 110 of the main body even though the skin receives an external pressure or a pressure after being inserted into the hypoderm, and thus the spiral elastic support 130 may reinforce a recoiling force of the main body. Meanwhile, the spiral elastic support 130 may also be located out of the main body 100 to surround an outer circumference of the main body 100. If the spiral elastic support 130 is formed to surround an outer circumference of the main body, the spiral elastic support 130 may give a buffering action against a pressure applied to the main body to support the main body. In addition, the spiral elastic support 130 may be made of the same material as the main body 100.

In addition, in addition to the above role of reinforcing a recoiling force, the spiral elastic support 130 allow the filler for removing wrinkles according to the present disclosure to be easily applied to a curved portion when being inserted into the hypoderm in a curved form since the spiral structure of the spiral elastic support 130 may be adjusted more densely or sparsely according to a curvature.

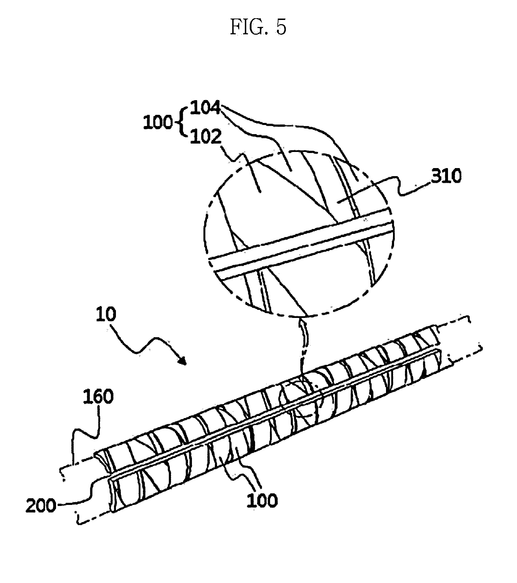

Fig. 5 is a perspective view showing a filler for removing wrinkles, in which at least two rows of band-type partial bodies are alternately entangled according to a fifth embodiment of the present disclosure.

As shown in Fig. 5, the wrinkle removing filler 10 according to the fifth embodiment of the present disclosure includes a main body 100 having an elongated tubular shape having at least two rows of band-type partial bodies 102, 104 spirally rolled alternately, and a longitudinal cleft 200 formed through the main body 100 in the length direction.

In the wrinkle removing filler 10 of the fifth embodiment, the elongated tubular main body 100 is formed by alternately entangling at least two rows of band-type partial bodies 102, 104, made of the same material of the main body 100 of the wrinkle removing filler 10, along a surface of an elongated core member 160 having a circular section (or, a hexagonal section) and a desired hollow diameter, and then the core member 160 is removed so that the hollow is formed in the partial bodies 102, 104 in the length direction. In addition, gaps among the plural rows of partial bodies 102, 104 (including gaps formed at cross points of the plural rows of partial bodies) may form through holes 310.

Different from the above, various methods may also be used, and for example the elongated tubular main body 100 may be formed by alternately entangling the plural rows of partial bodies 102, 104 without using a separate core member.

In addition, even though Fig. 5 illustrates that the wrinkle removing filler 10 according to the fifth embodiment of the present disclosure is formed by using two rows of partial bodies 102, 104, namely the first partial body 102 and the second partial body 104, the present disclosure is not limited thereto, and the elongated tubular main body 100 may be formed by spirally winding plural rows of band-type to be alternately entangled, as obvious to those skilled in the art.

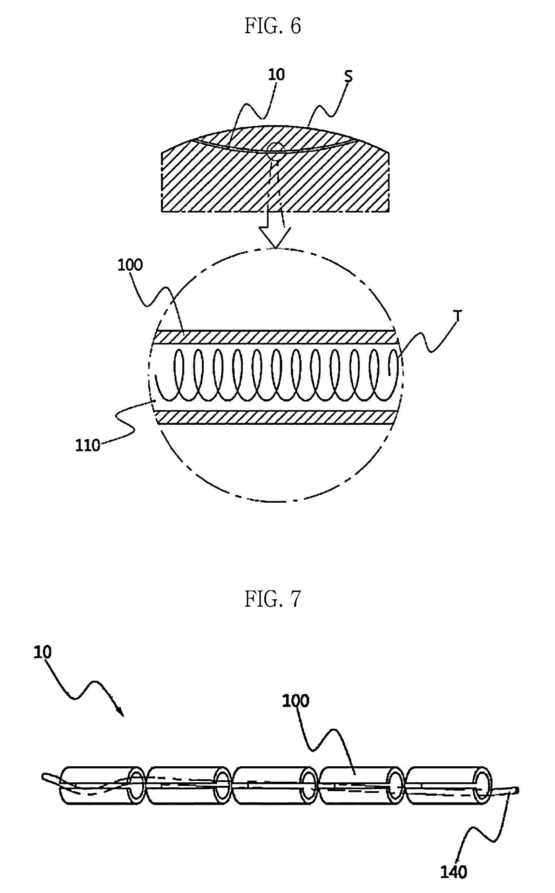

Fig. 6 is a cross-sectional view showing that fibrous tissues T are formed in a hollow of the filler for removing wrinkles according to the present disclosure, which is inserted into the hypoderm.

Referring to Fig. 6, a process of removing wrinkles by using the wrinkle removing filler according to the present disclosure will be described as follows. One terminal of the filler for removing wrinkles according to the present disclosure is threaded or connected to a tip of a needle for plastic surgery, or a needle for intravenous injection is inserted into a tube simply.

First, a wrinkle portion to be treated is marked, and an anesthesia salve is applied thereto. After that, a needle is inserted into the hypoderm, particularly between the subcutaneous layer and the dermis layer or at the fat layer, at one end of the marked portion, and is pulled out at the other end so that the wrinkle removing filler is placed over the marked portion. After that, an exposed portion out of the skin S is cut.

If so, the hollow 110 of the wrinkle removing filler 10 inserted into the hypoderm is filled with blood, red blood cells, white blood cells, blood platelet, fibroblast, myofibroblast or the like. The fibroblast comes to the maximum three to five days after the surgery, and the myofibroblast comes to the maximum five to fifteen days after the surgery. After that, collagen is formed at the fibroblast and remains as fibrous tissues T, and the fibrous tissues T gives an effect of swelling concave or caved portions due to wrinkles.

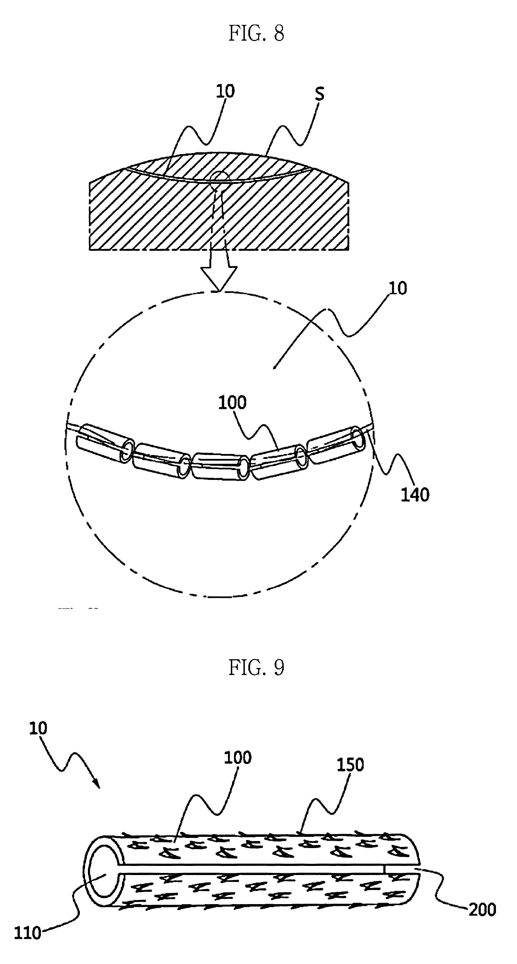

Fig. 7 is a perspective view showing a filler for removing wrinkles, in which a plurality of short main bodies respectively having a longitudinal cleft are interconnected by a connector according to a sixth embodiment of the present disclosure, and Fig. 8 is a cross-sectional view showing that the filler for removing wrinkles according to the sixth embodiment of the present disclosure is inserted into the hypoderm for plastic surgery.

As shown in Fig. 7, the wrinkle removing filler 10 according to the sixth embodiment of the present disclosure includes a short and thin tubular main body 100 having a hollow formed therein, a longitudinal cleft 200 formed through the main body in the length direction from an outer circumference of the main body to an inner circumference thereof, and a connector 140 installed in a plurality of short tubular main bodies arranged to have hollows adjacent to each other so that the hollows are connected through the main body in the length direction.

Even when a plurality of short main bodies are connected by a connector as described above, cells may easily move through the entire wrinkle removing filler which communicate with each other by a longitudinal cleft formed in each main body, thereby ensuring uniform and rapid formation of fibrous tissues.

When the wrinkle removing filler 10 in which a plurality of short main bodies 100 are connected by the connector 140 as shown in Fig. 8 is inserted into the hypoderm in a curved form, each of the plurality of short main bodies serves as a joint of each segment forming the curved form, thereby ensuring easier insertion. In addition, after the insertion, the main bodies may naturally maintain their bent state, thereby reducing an influence caused by an external force or pressure applied to the skin.

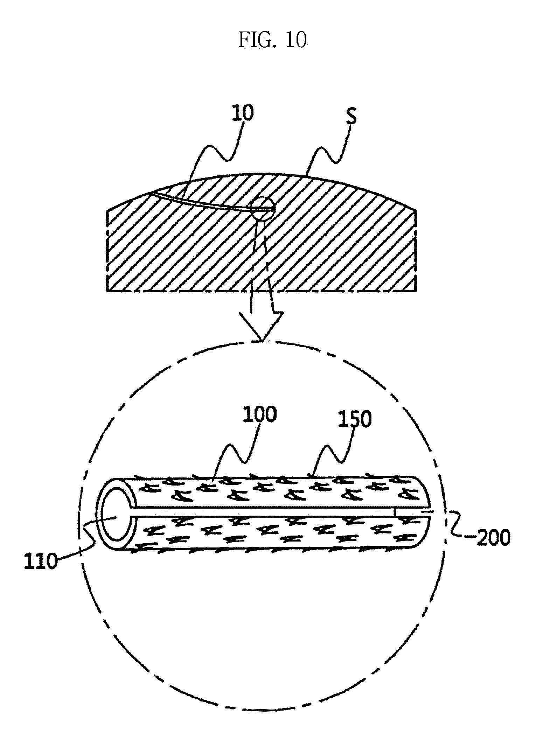

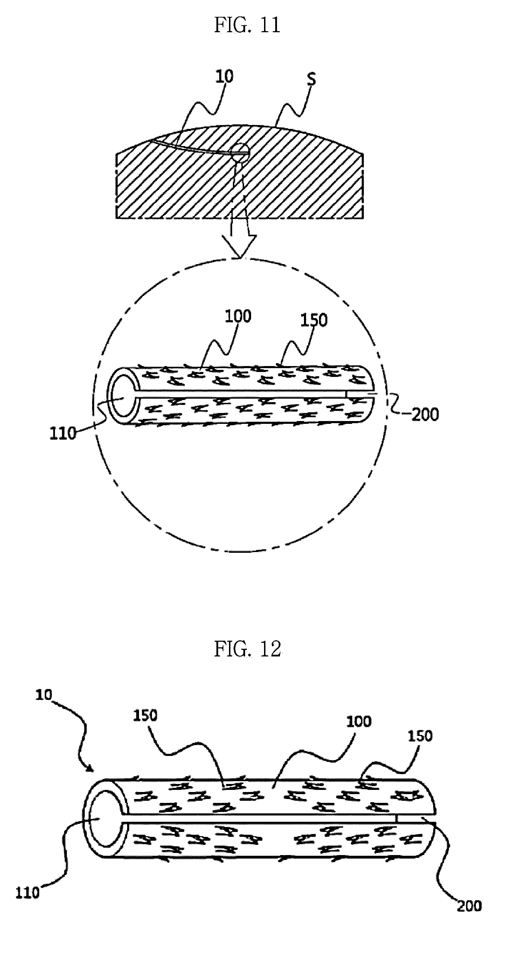

Fig. 9 is a perspective view showing a filler for removing wrinkles, in which an elastic cog inclined to a side is formed at a surface of the main body according to a seventh embodiment of the present disclosure, Fig. 10 is a cross-sectional view showing a plastic surgery state while the filler for removing wrinkles according to the seventh embodiment of the present disclosure is being inserted into the hypoderm, and Fig. 11 is a cross-sectional view showing that an elastic cog is hooked to the fibrous tissues T after the filler for removing wrinkles according to the seventh embodiment of the present disclosure is completely inserted into the hypoderm.

As shown in Fig. 9, the wrinkle removing filler 10 according to the seventh embodiment of the present disclosure includes an elongated integrated tubular main body 100, a longitudinal cleft 200 formed through the main body 100 in the length direction, and an elastic cog 150 formed by partially cutting a surface of the main body at an outer circumference thereof.

The elastic cog 150 deviates to one side along the length direction of the main body at the outer circumference of the main body 100 to protrude out of the outer circumference of the main body, and thus may be more stably fixed to neighboring tissues of the hypoderm into which the filler is inserted.

As shown in Fig. 10, the wrinkle removing filler 10 according to the seventh embodiment of the present disclosure may be smoothly inserted into the hypoderm since the elastic cog 150 maintains a bent state (a state in which the elastic cog is received in a processing groove formed by caving a part of the outer circumferential surface) while the wrinkle removing filler 10 is being inserted into the hypoderm. However, as shown in Fig. 11, if the wrinkle removing filler 10 is completely inserted into the hypoderm for surgery, the elastic cog 150 restores its original protruding state (see Fig. 9) as deviating to one side from the main body 100 due to its elasticity.

The elastic cog 150 restoring to its original state is hooked by neighboring tissues T to prevent the wrinkle removing filler 10 from retrieving in the hypoderm, thereby enhancing the fixedness against the hypoderm.

Fig. 12 is a perspective view showing a filler for removing wrinkles, in which an elastic cog having a decalcomanias shape symmetric based on a center portion of the main body is formed at a surface of the main body according to an eighth embodiment of the present disclosure.

As shown in Fig. 12, the wrinkle removing filler 10 according to the eighth embodiment of the present disclosure includes an elongated integrated tubular main body 100, a longitudinal cleft 200 formed through the main body 100 in the length direction, and an elastic cog 150 formed by partially cutting a surface of the main body at an outer circumference thereof to have an acute angle with a decalcomanias shape symmetric based on a center portion of the main body.

Since the elastic cogs 150 have a decalcomanias arrangement symmetric based on the center portion of the elongated main body, the elastic cogs 150 are provided symmetrically to have an acute angle based on the center portion.

Accordingly, when passing in an advancing direction, the elastic cog 150 moves in a forwarding direction since a front portion of the main body 100 moves forwards in the subcutaneous layer. However, after advancing, the main body is pulled in an opposite direction so that the forwarding elastic cog turns to an opposite direction.

Meanwhile, even though the elastic cog 150 located in an opposite side based on the center portion is applied in an opposite direction at an early stage, when the main body is pulled in a direction opposite to the early stage, the elastic cog 150 has a forwarding direction. However, the elastic cogs in a front portion are spread in an opposite direction to prohibit advancing.

Therefore, by inserting the main body 100 into the subcutaneous layer, which allows easy insertion, and then reciprocating the main body 100 forwards and rearwards one or two times, the main body may be fixed. In other words, the elastic cog 150 formed in opposite directions based on the center portion play an important role in fixing the main body 100. This configuration may be used for lifting and fixing droop tissues or more securely fixing the inserted main body. In particular, since tissues formed in the hollow and tissues out of the main body are bound with a single connector, the lifting effect may be more enhanced.

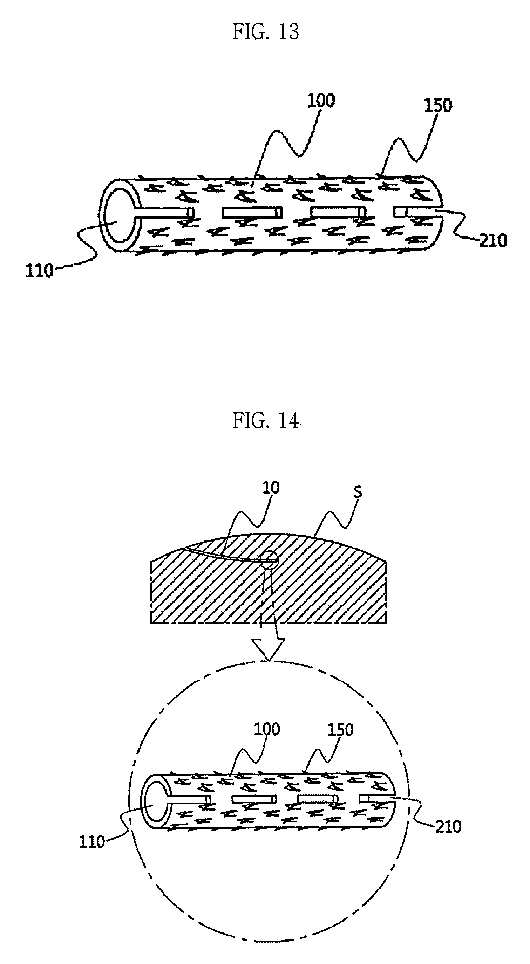

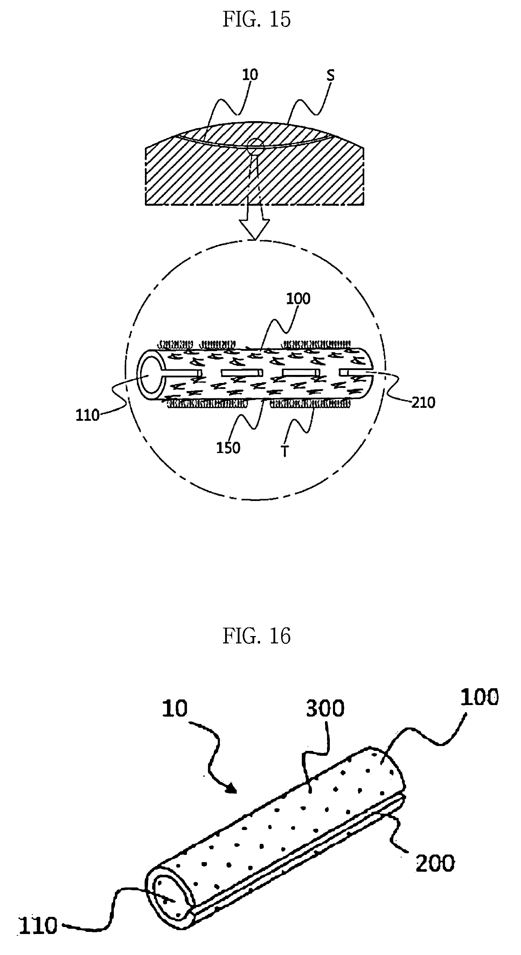

Fig. 13 is a perspective view showing a filler for removing wrinkles, in which separated longitudinal clefts and an elastic cog are formed together according to a ninth embodiment of the present disclosure, Fig. 14 is a cross-sectional view showing a plastic surgery state while the filler for removing wrinkles according to the ninth embodiment of the present disclosure is being inserted into the hypoderm, and Fig. 15 is a cross-sectional view showing that an elastic cog is hooked to the fibrous tissues T after the filler for removing wrinkles according to the ninth embodiment of the present disclosure is completely inserted into the hypoderm.

As shown in Fig. 13, the wrinkle removing filler 10 according to the ninth embodiment of the present disclosure includes an elongated integrated tubular main body 100, separated longitudinal clefts 210 formed through the main body in the length direction and spaced apart from each other with at least two gaps to form a moving passage of cells placed at neighboring tissues of the main body 100 so that the cells move into the main body 100 and form fibrous tissues, and an elastic cog 150 formed by partially cutting a surface of the main body at an outer circumference thereof.

As described above in relation to the seventh embodiment, the elastic cog 150 deviates to one side along the length direction of the main body at the outer circumference of the main body 100 to protrude out of the outer circumference of the main body.

As shown in Fig. 14, the wrinkle removing filler 10 according to the ninth embodiment of the present disclosure may be smoothly inserted into the hypoderm since the elastic cog 150 maintains a bent state while the wrinkle removing filler 10 is being inserted into the hypoderm. However, as shown in Fig. 15, if the wrinkle removing filler 10 is completely inserted into the hypoderm for surgery, the elastic cog 150 restores its original protruding state as deviating to one side from the main body 100 due to its elasticity.

The elastic cog 150 restoring to its original state as described above is hooked by neighboring tissues T to prevent the wrinkle removing filler 10 from retrieving in the hypoderm, thereby enhancing the fixedness against the hypoderm, substantially identical to the seventh embodiment.

Fig. 16 is a perspective view showing a filler for removing wrinkles, in which a longitudinal cleft and a through hole are formed together according to a tenth embodiment of the present disclosure.

As shown in Fig. 16, the wrinkle removing filler 10 according to the tenth embodiment of the present disclosure includes an elongated integrated tubular main body 100, a longitudinal cleft 200 formed through the main body in the length direction to form a moving passage of cells placed at neighboring tissues of the main body so that the cells move into the main body and form fibrous tissues, and at least one through hole 300 formed through the entire surface of the main body.

Accordingly, the through hole 300 as well as the longitudinal cleft 200 forms a passage through which fibroblast or the like of neighboring tissues enters the hollow 110 of the main body to newly form fibrous tissues such as elastic fibrous tissues and collagen tissues.

The through hole 300 have any shape such as circular, triangular, rectangular, octagonal, trapezoidal, or diamond shape, and a plurality of through holes 300 may be regularly or irregularly arranged at the outer circumference of the main body 100. In addition, the through hole 300 may have a diameter of 40 to 500 µm in case of a circular shape or a circularly-converted diameter of 40 to 500 µm with the same sectional area. If the diameter is too great, a supporting force against neighboring tissues is lost disadvantageously.

At this time, the main body and the longitudinal cleft are substantially identical to those of the first embodiment and are thus not described in detail here.

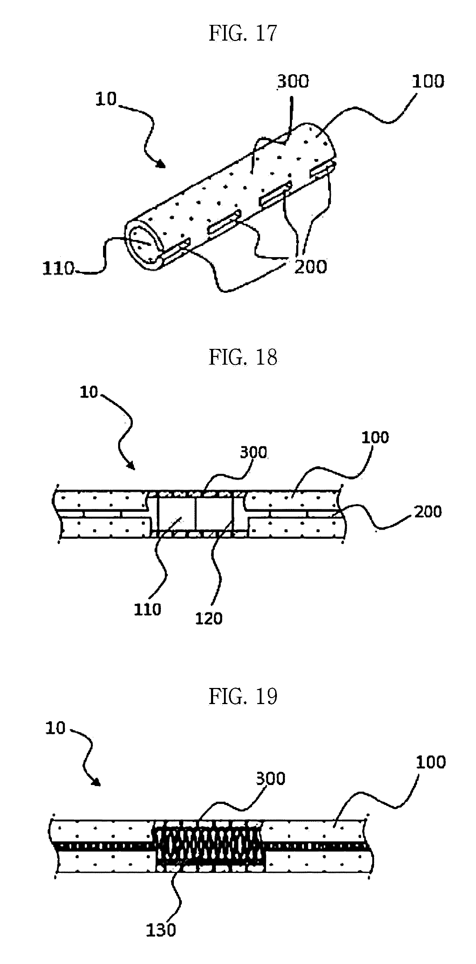

Fig. 17 is a perspective view showing a filler for removing wrinkles, in which separated longitudinal clefts and a through hole are formed together according to an eleventh embodiment of the present disclosure.

As shown in Fig. 17, the wrinkle removing filler 10 according to the eleventh embodiment of the present disclosure includes an elongated integrated tubular main body 100 and allowing surgery through the hypoderm, separated longitudinal clefts 210 formed through the main body in the length direction and spaced apart from each other with at least two gaps to form a moving passage of cells placed at neighboring tissues of the main body so that the cells move into the main body and form fibrous tissues, and at least one through hole 300 formed through the entire surface of the main body.

At this time, the separated longitudinal clefts 210 is substantially identical to the separated longitudinal clefts 210 of the second embodiment, and the through hole 300 is also substantially identical to that of the tenth embodiment. Thus, they are not described in detail here.

Fig. 18 is a perspective view showing a filler for removing wrinkles, in which a longitudinal cleft, a barrier and a through hole are formed together according to a twelfth embodiment of the present disclosure.

As shown in Fig. 18, the wrinkle removing filler 10 according to the twelfth embodiment of the present disclosure includes an elongated integrated tubular main body 100, a longitudinal cleft 200 formed through the main body 100 in the length direction, a plurality of barriers 120 formed in the hollow 110 of the main body, and at least one through hole 300 formed through the entire surface of the main body.

At this time, the barrier 120 gives a recoiling force to support the filler inserted into the hypoderm so that the hollow in the filler is maintained, substantially identical to that of the third embodiment, and the through holes 300 are formed through the entire area of the main body to ensure more uniform and easier formation of fibrous tissues.

Fig. 19 is a perspective view showing a filler for removing wrinkles, in which a longitudinal cleft, a spiral elastic support and a through hole are formed together according to a thirteenth embodiment of the present disclosure.

As shown in Fig. 19, the wrinkle removing filler 10 according to the thirteenth embodiment of the present disclosure includes an elongated integrated tubular main body 100, a longitudinal cleft 200 formed through the main body 100 in the length direction, a spiral elastic support 130 formed in the hollow 110 of the main body, and at least one through hole 300 formed through the entire surface of the main body.

At this time, the spiral elastic support 130 is substantially identical to that of the fourth embodiment, and the through hole 300 is formed through the entire surface of the main body to ensure more uniform and easier formation of fibrous tissues. Moreover, the through hole 300 is substantially identical to that of the tenth embodiment and thus is not described in detail here.

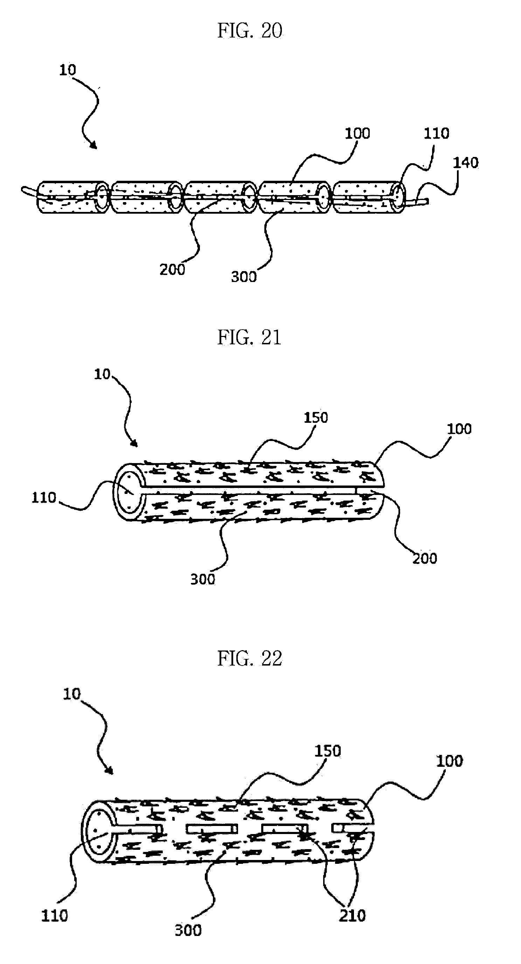

Fig. 20 is a perspective view showing a filler for removing wrinkles, in which a plurality of short main bodies respectively having a longitudinal cleft and a through hole together are interconnected by a connector according to a fourteenth embodiment of the present disclosure.

As shown in Fig. 20, the wrinkle removing filler 10 according to the fourteenth embodiment of the present disclosure includes a short and thin tubular main body 100 having a hollow formed therein, a longitudinal cleft 200 formed through the main body in the length direction from an outer circumference of the main body to an inner circumference thereof, a connector 140 installed in a plurality of short tubular main bodies arranged to have hollows adjacent to each other so that the hollows are connected through the main body in the length direction, and at least one through hole 300 formed through the entire surface of the main body.

At this time, since a plurality of short main bodies are connected by the connector, when the filler is inserted into the hypoderm in a curved form, each of the plurality of short main bodies serves as a joint of each segment forming the curved form, thereby ensuring easier insertion. In addition, after the insertion, the main bodies may naturally maintain their bent state, thereby reducing an influence caused by an external force or pressure applied to the skin, substantially identical to the sixth embodiment. In addition, the through hole 300 is substantially identical to that of the tenth embodiment and thus is not described in detail here.

Fig. 21 is a perspective view showing a filler for removing wrinkles, in which a longitudinal cleft, an elastic cog and a through hole are formed together according to a fifteenth embodiment of the present disclosure.

As shown in Fig. 21, the wrinkle removing filler 10 according to the fifteenth embodiment of the present disclosure includes an elongated integrated tubular main body 100, a longitudinal cleft 200 formed through the main body 100 in the length direction, an elastic cog 150 formed by partially cutting a surface of the main body at an outer circumference thereof, and at least one through hole 300 formed through the entire surface of the main body.

The elastic cog 150 deviates to one side along the length direction of the main body 100 to protrude out of the outer circumference of the main body, and thus may be more stably fixed to neighboring tissues of the hypoderm into which the filler is inserted, substantially identical to the seventh embodiment. Accordingly, the wrinkle removing filler having the elastic cog 150 is also inserted into the hypoderm in substantially the same way as the seventh embodiment, which is thus not described in detail here. In addition, the through hole 300 is substantially identical to that of the tenth embodiment and thus is not described in detail here.

Fig. 22 is a perspective view showing a filler for removing wrinkles, in which separated longitudinal clefts, an elastic cog and a through hole are formed together according to a sixteenth embodiment of the present disclosure.

As shown in Fig. 22, the wrinkle removing filler 10 according to the sixteenth embodiment of the present disclosure includes an elongated integrated tubular main body 100, separated longitudinal clefts 210 formed through the main body in the length direction and spaced apart from each other with at least two gaps to form a moving passage of cells placed at neighboring tissues of the main body so that the cells move into the main body 100 and form fibrous tissues, an elastic cog 150 formed by partially cutting a surface of the main body at an outer circumference thereof, and at least one through hole 300 formed through the entire surface of the main body.

The elastic cog 150 deviates to one side along the length direction of the main body at the outer circumference of the main body 100 to protrude out of the outer circumference of the main body, and thus may be more stably fixed to neighboring tissues of the hypoderm into which the filler is inserted, as described above in relation to the seventh embodiment. In addition, the through hole 300 is substantially identical to that of the tenth embodiment and thus is not described in detail here.

In the filler for removing wrinkles according to the first to sixteenth embodiments of the present disclosure configured as above, a contrast medium may be partially or entirely applied to an outside of the main body 100 or included in the main body 100 to trace a location of the wrinkle removing filler or check the degree of decomposition thereof.

In addition, in the filler for removing wrinkles according to the first to sixteenth embodiments of the present disclosure, a growth factor for promoting formation of fibrous tissues may be coated to an outer circumference or inner circumference of the main body 100 or included in the main body 100.

At this time, the growth factor may use a platelet-derived growth factor (PDGF), an angiogenesis factor (AGF), a fibroblast growth factor (FGF), a transforming growth factor-α (TGF-α), a transforming growth factor-β (TGF-β), an epidermal growth factor (EGF), a connective tissue growth factor (CTGF), a vascular endothelial growth factor (VEGF) or the like, without being limited thereto.

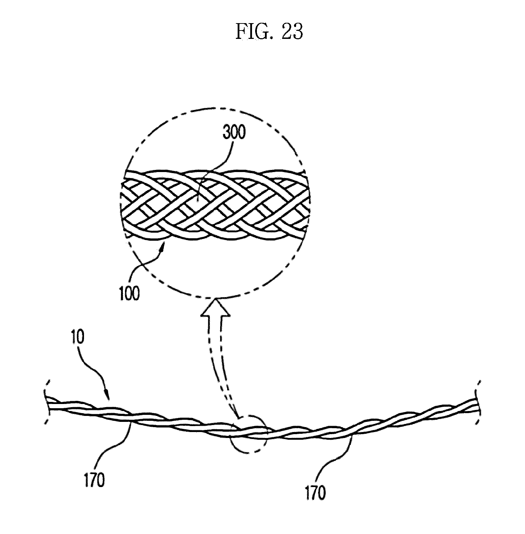

Referring to Fig. 23 which is a perspective view and a partially enlarged view showing a filler for removing wrinkles, in which at least two rows of band-type partial bodies are alternately entangled according to a seventeenth embodiment of the present disclosure, since at least two rows of band-type partial bodies 102, 104 are alternately entangled and spirally wound to form a main body 100 having an elongated tubular shape, through holes 300 are formed through the main body 100 between the partial bodies 102, 104. Therefore, since the through holes 300 are formed in a region of the main body 100 except for the partial bodies 102, 104, fibrous tissues may be regenerated at a more regular rate in the entire main body.

In the main body 100 of the wrinkle removing filler 10 as described above, concave portions 170 spaced apart from each other may be formed as shown in Fig. 23. The concave portion 170 reinforces a supporting force of the main body 100. Referring to Fig. 23 showing a graph showing a strength comparison experiment result, it is assumed that a pressure is applied to outsides of main bodies respectively having a diameter of 1.0 mm and a diameter of 0.6 mm. In this case, a main body having a diameter of 1.0 mm in which the concave portion 170 is formed maintains its shape even though a pressure of about 40 gf/cm² is applied thereto. However, a main body not having the concave portion 170 does not maintain its shape if a pressure greater than about 10 gf/cm² is applied thereto. In addition, a main body having a diameter of 0.6 mm in which the concave portion 170 is formed maintains its shape at a pressure of about 25 gf/cm², but a main body not having the concave portion 170 maintains its shape just up to about 15 gf/cm². Therefore, it is more desirable that the concave portion 170 is formed in the main body 100.

The concave portion 170 may be formed in various ways. If the main body 100 is made of polymer, it is easy to form the concave portion 170 by shrinking the main body through thermal treatment. The partial bodies 102, 104 described above may use a suture as shown in Fig. 23 or another separate member. If the suture is used, a suture made of biodegradable polymer and a suture made of non-biodegradable may be used as a mixture.

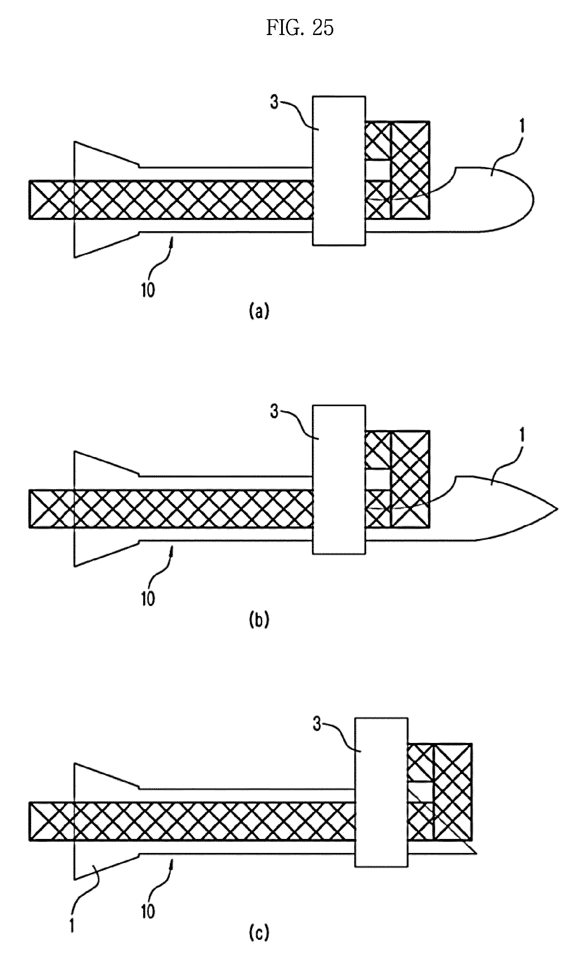

The wrinkle removing filler 10 according to the seventeenth embodiment is coupled to a needle as shown in Portions (a), (b) and (c) of Fig. 25. Here, one end of the wrinkle removing filler 10 is placed through a hole formed at both sides of a needle 1 of various shapes, and one end of the wrinkle removing filler 10 passing through the needle 1 is bent using a separate fixture 3 such as sponge so as to be coupled and fixed to the needle 1.

In addition, tissue creation material such as retinoic acid, retinol derivatives, vitamin A or the like may be applied, coated or absorbed to the main body 100 or the partial bodies 102, 104 of the main body 100. Moreover, depending on embodiments, inflammation inducing promoters such as catgut thread material, tissue creation promoters such as platelet rich plasma (PRP), tissue creation restrainers for reducing excessive tissue creation, or tissue creation inhibitors such as an anti-inflammatory agent may also be applied, coated or absorbed.

The above embodiments are just to illustrate the present disclosure for better understanding, and the present disclosure is not limited thereto. Various changes or modifications can be made within the scope of the present disclosure by those skilled in the art without departing from the scope of the appended claims.

## Claims

1. A filler for removing wrinkles, comprising:
an elongated integrated tubular main body having a hollow formed therein and allowing surgery through the hypoderm; and
a longitudinal cleft formed through the main body in the length direction to form a moving passage of cells placed at neighboring tissues of the main body so that the cells move into the main body and form fibrous tissues.

2. A filler for removing wrinkles, comprising:
an elongated integrated tubular main body having a hollow formed therein and allowing surgery through the hypoderm; and
separated longitudinal clefts formed through the main body in the length direction and spaced apart from each other with at least two gaps to form a moving passage of cells placed at neighboring tissues of the main body so that the cells move into the main body and form fibrous tissues.

3. A filler for removing wrinkles, comprising:
an elongated tubular main body having at least two rows of band-type partial bodies spirally rolled alternately; and
a longitudinal cleft formed through the main body in the length direction.

4. A filler for removing wrinkles, comprising:
a short and thin tubular main body having a hollow formed therein;
a longitudinal cleft formed through the main body in the length direction from an outer circumference of the main body to an inner circumference thereof; and
a connector installed in a plurality of short and thin tubular main bodies arranged to have hollows adjacent to each other so that the hollows are connected through the main body in the length direction.

5. A filler for removing wrinkles, comprising:
an elongated integrated tubular main body having a hollow formed therein;
a longitudinal cleft formed through the main body in the length direction; and
an elastic cog formed by partially cutting a surface of the main body at an outer circumference thereof.

6. A filler for removing wrinkles, comprising:
an elongated integrated tubular main body;
a longitudinal cleft formed through the main body in the length direction; and
an elastic cog formed by partially cutting a surface of the main body at an outer circumference thereof to have an acute angle with a decalcomanias shape symmetric based on a center portion of the main body.

7. A filler for removing wrinkles, comprising:
an elongated integrated tubular main body having a hollow formed therein;
separated longitudinal clefts formed through the main body in the length direction and spaced apart from each other with at least two gaps to form a moving passage of cells placed at neighboring tissues of the main body so that the cells move into the main body and form fibrous tissues; and
an elastic cog formed by partially cutting a surface of the main body at an outer circumference thereof.

8. A filler for removing wrinkles, comprising:
an elongated tubular main body having at least two rows of band-type partial bodies spirally rolled alternately; and
a plurality of through holes formed through outer and inner surfaces of the main body,
wherein the through holes are formed between the partial bodies.

9. The filler for removing wrinkles according to claim 1, further comprising:
a plurality of barriers formed in the hollow of the main body.

10. The filler for removing wrinkles according to claim 1, further comprising:
a spiral elastic support formed in the hollow of the main body.

11. The filler for removing wrinkles according to claim 1, further comprising:
a spiral elastic support surrounding the main body.

12. The filler for removing wrinkles according to claim 8,
wherein the main body has concave portions spaced apart from each other.

13. The filler for removing wrinkles according to claim 12,
wherein the concave portions are formed by shrinking the main body.

14. The filler for removing wrinkles according to any one of claims 1 to 13,
wherein the main body has a circular cross section with a diameter of 0.6 to 3.0 mm, and the hollow has a diameter of 0.5 to 2.8 mm.

15. The filler for removing wrinkles according to any one of claims 1 to 13,
wherein the main body has a polygonal cross section.

16. The filler for removing wrinkles according to any one of claims 1 to 7 and 9 to 11,
wherein the longitudinal cleft has a gap distance of 0.1 to 2.0 mm.

17. The filler for removing wrinkles according to any one of claims 1 to 7 and 9 to 11,
wherein the longitudinal cleft has a gap distance which is 1/8 to 1/4 of a circularly-converted circumferential length of a section of the main body.

18. The filler for removing wrinkles according to any one of claims 1 to 13,
wherein the main body is made of elastic material.

19. The filler for removing wrinkles according to claim 18,
wherein the main body is made of a biodegradable polymer selected from the group consisting of hyaluronic acid (HA), polylactic acid (PLA), polyglyco-lactic acid (PGLA) and polydioxanone (PDS).

20. The filler for removing wrinkles according to claim 18,
wherein the main body is made of a non-biodegradable polymer selected from the group consisting of nylon, silicon, polypropylene, polypropylethylene and Teflon.

21. The filler for removing wrinkles according to claim 18,
wherein the main body has a double-layer structure in which inner and outer layers of the main body are made of different materials.

22. The filler for removing wrinkles according to claim 21,
wherein the inner layer is made of hard material, and the outer layer is made of soft material.

23. The filler for removing wrinkles according to claim 21,
wherein the inner layer is made of absorptive material, and the outer layer is made of non-absorptive material.

24. The filler for removing wrinkles according to claim 21,
wherein the inner and outer layers are made of absorptive materials with different absorption rates.

25. The filler for removing wrinkles according to any one of claims 1 to 7,
wherein the main body has at least one through hole formed through the entire surface thereof.

26. The filler for removing wrinkles according to claim 25,
wherein the through hole has a diameter of 40 to 500 µm in case of a circular shape or a circularly-converted diameter of 40 to 500 µm with the same sectional area.

27. The filler for removing wrinkles according to any one of claims 1 to 13,
wherein a contrast medium is partially or entirely coated to an outside of the main body.

28. The filler for removing wrinkles according to any one of claims 1 to 13,
wherein a growth factor for promoting formation of fibrous tissues is coated to an outer circumference or inner circumference of the main body.

29. The filler for removing wrinkles according to claim 25,
wherein a contrast medium is partially or entirely coated to an outside of the main body.

30. The filler for removing wrinkles according to claim 25,
wherein a growth factor for promoting formation of fibrous tissues is coated to an outer circumference or inner circumference of the main body.

31. The filler for removing wrinkles according to claim 25,
wherein any one of tissue creation material, inflammation inducing promoter, tissue creation restrainer and tissue creation inhibitor is coated, applied or absorbed to the main body.
